(19) Europäisches Patentamt
European Patent Office
Office européen des brevets

(11) EP 0 773 217 A1

(12) # EUROPEAN PATENT APPLICATION

(43) Date of publication:
**14.05.1997 Bulletin 1997/20**

(51) Int Cl.[6]: **C07D 333/56**, A61K 31/34, A61K 31/38, A61K 31/40

(21) Application number: **96307955.3**

(22) Date of filing: **04.11.1996**

(84) Designated Contracting States:
**AT BE CH DE DK ES FI FR GB GR IE IT LI LU NL PT SE**
Designated Extension States:
**AL LT LV RO SI**

(30) Priority: **07.11.1995 US 6350**

(71) Applicant: **ELI LILLY AND COMPANY**
**Indianapolis, Indiana 46285 (US)**

(72) Inventors:
- **Dantzig, Anne Hollins**
  **Crawfordsville, Indiana 47933 (US)**
- **Grese, Timothy Alan**
  **Indianapolis, Indiana 46236 (US)**
- **Norman, Bryan Hurst**
  **Indianapolis, Indiana 46236 (US)**
- **Palkowitz, Alan David**
  **Carmel, Indiana 46032 (US)**
- **Sluka, James Patrick**
  **Greenwood, Indiana 46143 (US)**
- **Starling, James Jacob**
  **Carmel, Indiana 46033 (US)**
- **Winter, Mark Alan**
  **Indianapolis, Indiana 46220 (US)**

(74) Representative: **Hudson, Christopher Mark**
**Lilly Industries Limited**
**European Patent Operations**
**Erl Wood Manor**
**Windlesham Surrey GU20 6PH (GB)**

(54) **Benzothiphene derivatives for treating resistant tumors**

(57) This invention provides a series of substituted benzo[b]thiophenes useful in reversing multidrug resistance in a resistant neoplasm. The present invention also provides methods for reversing the multidrug resistance in a resistant neoplasm by treating a mammal in need of said treatment with a substituted benzothiophene. This invention also provides methods for treating neoplasms in a mammal which comprises administering to a mammal in need of this treatment a substituted benzothiophene in combination with an oncolytic agent.

EP 0 773 217 A1

## Description

Along with surgery and radiotherapy, chemotherapy continues to be an effective therapy for many cancers. In fact, several types of cancer are now considered to be curable by chemotherapy and include Hodgkin's disease, large cell lymphoma, acute lymphocytic leukemia, testicular cancer and early stage breast cancer. Other cancers such as ovarian cancer, small cell lung and advanced breast cancer, while not yet curable, are exhibiting positive response to combination chemotherapy.

One of the most important unsolved problems in cancer treatment is drug resistance. Drug resistance includes both intrinsic resistance at the time of treatment using chemotherapy and acquired drug resistance. This problem is a reason for the added importance of combination chemotherapy, as the therapy both has to avoid the emergence of resistant cells and to kill pre-existing cells which are already drug resistant.

Anthracyclines represent an important class of oncolytic agents. Doxorubicin, an anthracycline, which is also known in the art as ADRIAMYCIN™, is a drug of choice in the clinical management of breast cancer. Therapy with anthracyclines such as doxorubicin is complicated by the appearance of the anthracycline resistant phenotype which limits or negates the oncolytic activity of doxorubicin.

Taxol (PACLITAXEL™) is an antineoplastic taxane derivative originally isolated from Taxus spp. yew tree. This compound, and later derivatives thereof, are useful in the treatment of metastatic ovarian carcinoma which is refractory to first-line chemotherapy.

Topoisomerase inhibitors represent a further class of oncolytic agents. Epipodophyllotoxins such as ETOPOSIDE® and TENIPOSIDE® are topoisomerase inhibitors which are useful in the therapy of neoplasms of the testis, small-cell lung and other lung, breast, Hodgkin's disease, non-Hodgkin's lymphomas, acute granulocytic leukemia and Karposi's sarcoma. The therapeutic utility of the epipodophylotoxins is limited by the appearance of the epipodophyllotoxin resistant phenotype.

One form of multi-drug resistance (MDR) is mediated by a membrane bound 170-180 kD energy-dependent efflux pump designated as P-glycoprotein (P-gp). P-glycoprotein has been shown to play a major role in the intrinsic and acquired resistance of a number of human tumors against hydrophobic, natural product drugs. Drugs that act as substrates for and are consequently detoxified by P-gp include the vinca alkaloids (vincristine and vinblastine), anthracyclines (Adriamycin), and epipodophyllotoxins (etoposide). While P-gp associated MDR is a major determinant in tumor cell resistance to chemotherapeutic agents, it is clear that the phenomenon of MDR is multifactorial and involves a number of different mechanisms. One such alternative pathway for resistance to anthracyclines involves the emergence of a 190 kD protein (p190)that is not P-gp. See, T. McGrath, et al., Biochemical Pharmacolology, 38:3611 (1989). The protein p190 is not found exclusively on the plasma membrane but rather appears to be localized predominantly in the endoplasmic reticulum. See, e.g., D. Marquardt, and M.S. Center, Cancer Research, 52:3157 (1992).

The protein p190 possesses a nucleotide binding domain that is homologous with the ATP binding site of P-gp. See, D. Marquardt, et al., Cancer research, 50:1426 (1990). The mechanism(s) utilized by p190 to confer resistance to ADRIAMYCIN™ is not well understood but may involve the intracellular redistribution of ADRIAMYCIN™ away from the nucleus. See, D. Marquardt and M.S. Center, supra. ADRIAMYCIN™ is an inhibitor of topoisomerase II [W.T. Beck, Bulletins in Cancer, 77:1131 (1990)] which is an enzyme involved in DNA replication. Redistribution of ADRIAMYCIN™ away from the nucleus would therefore be an important component in cellular resistance to this drug. The studies published to date on p190 have utilized cell lines selected in vitro for resistance to ADRIAMYCIN™. T. McGrath, et al., supra; D. Marquardt and M.S. Center, supra; and D. Marquardt, et al., Cancer Research, supra.

The association of p190 with drug resistance was made by sodium dodecyl sulfate polyacrylamide gel electrophoresis (SDS-PAGE) of radioactive extracts prepared from Adriamycin-resistant HL60/Adr human leukemia cells labeled with 8-azido-alpha[$^{32}$P]ATP. See, T. McGrath, et al., supra. The drug-resistance phenotype conferred by p190 is not limited to the anthracyclines. Epipodophyllotoxin resistance is linked to p190 expression. The $IC_{50}$'s of HL60/S cells treated with ADRIAMYCIN™ and ETOPOSIDE™ were 0.011 μg/ml and 0.39 μg/ml respectively. The $IC_{50}$'s for HL60/Adr cells (a HL60-derived cell line which is resistant to doxorubicin) treated with Adriamycin and Etoposide were 2.2 μg/ml and >10 μg/ml respectively. HL60/S and HL60/Adr cell lines do not express P-glycoprotein. HL60/Adr expresses p190. Thus, resistance to the anthracyclines and epipodophyllotoxins results from p190 expression.

It is, therefore, desirable to provide compounds which are useful for treating resistant neoplasms, the resistant pathway including p190, P-glycoprotein, or both.

This invention provides a method of reversing multidrug resistance in a multidrug resistant tumor in a mammal which comprising administering to a mammal in need thereof a multidrug resistance reversing amount of a compound of Formula I:

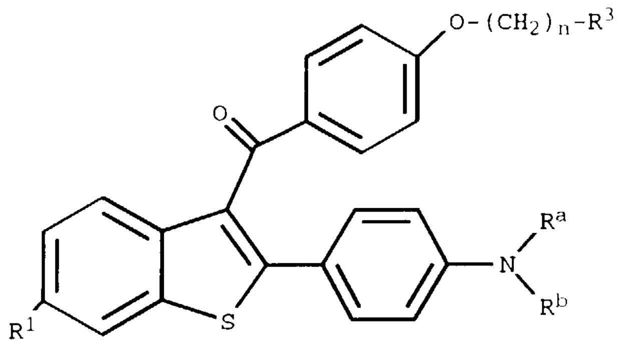

I

wherein:

$R^1$ is hydroxy, halo, hydrogen, $C_1$-$C_6$ alkoxy, or $C_1$-$C_6$ alkyl;

$R^a$ and $R^b$ are independently hydrogen, $C_1$-$C_6$ alkyl, -C(O)$R^c$, or -$SO_2$-$R^c$ , where $R^c$ is $C_1$-$C_6$ alkyl, halo, or trifluoromethyl;

n is 1-6;

$R^3$ is a group of the formula

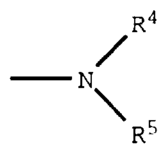

wherein $R^4$ and $R^5$ are independently $C_1$-$C_6$ alkyl or combine to form, along with the nitrogen to which they are attached, a heterocyclic ring selected from the group consisting of hexamethyleneiminyl, piperazinyl, heptamethyleneiminyl, imidazolinyl, piperidinyl, pyrrolidinyl, or morpholinyl;

or a pharmaceutically acceptable salt or solvate thereof.

The present invention also provides the novel compounds of Formula I as well as pharmaceutical compositions comprising a compound of Formula I in combination with one or more pharmaceutically acceptable carriers, diluents, or excipients therefor.

The present invention also provides methods for treating a susceptible neoplasm in a mammal which comprises administering a compound of Formula I in combination with an oncolytic agent.

In another embodiment, the present invention provides for pharmaceutical compositions comprising a compound of Formula I, in combination with an oncolytic, in addition to one or more pharmaceutically acceptable carriers, diluents, or excipients therefor.

In another embodiment, the present invention provides for the use of a compound of Formula I for the manufacture of a medicament for the treatment of multidrug resistance in a multidrug resistant tumor in a mammal.

In another embodiment, the present invention provides for the use of a compound of Formula I and one or more oncolytic agents for the manufacture of a medicament for the treatment of a susceptible neoplasm in a mammal.

The current invention concerns the discovery that a select group of substituted benzothiophenes, those of Formula I, are useful as in reversing multidrug resistance in a resistant neoplasm.

The terms and abbreviations used in the instant examples have their normal meanings unless otherwise designated. For example "°C" refers to degrees Celsius; "N" refers to normal or normality; "mmol" refers to millimole or millimoles; "g" refers to gram or grams; "ml" means milliliter or milliliters; "M" refers to molar or molarity; "MS" refers to mass spectrometry; "IR" refers to infrared spectroscopy; and "NMR" refers to nuclear magnetic resonance spectroscopy.

As used herein, the term "$C_1$-$C_6$ alkyl" refers to straight or branched, monovalent, saturated aliphatic chains of 1 to 6 carbon atoms and includes, but is not limited to, methyl, ethyl, propyl, isopropyl, butyl, isobutyl, t-butyl, pentyl,

isopentyl, and hexyl.

"$C_1$-$C_6$ alkoxy" represents a straight or branched alkyl chain having from one to six carbon atoms attached to an oxygen atom. Typical $C_1$-$C_6$ alkoxy groups include methoxy, ethoxy, propoxy, isopropoxy, butoxy, *t*-butoxy, pentoxy and the like. The term "$C_1$-$C_6$ alkoxy" includes within its definition the term "$C_1$-$C_4$ alkoxy".

"$C_1$-$C_6$ alkylidenyl" refers to a straight or branched, divalent, saturated aliphatic chains of 1 to 6 carbon atoms and includes, but is not limited to, methylenyl, ethylenyl, propylenyl, isopropylenyl, butylenyl, isobutylenyl, *t*-butylenyl, pentylenyl, isopentylenyl, hexylenyl, and the like. The term "$C_1$-$C_4$ alkylidenyl" is encompassed within the term "$C_1$-$C_6$ alkylidenyl".

The term "halo" encompasses chloro, fluoro, bromo and iodo.

The term "leaving group" as used herein refers to a group of atoms that is displaced from a carbon atom by the attack of a nucleophile in a nucleophilic substitution reaction. The term "leaving group" as used in this document encompasses, but is not limited to, activating groups.

The term "activating group" as used herein refers a leaving group which, when taken with the carbonyl (-C=O) group to which it is attached, is more likely to take part in an acylation reaction than would be the case if the group were not present, as in the free acid. Such activating groups are well-known to those skilled in the art and may be, for example, succinimidoxy, phthalimidoxy, benzotriazolyloxy, benzenesulfonyloxy, methanesulfonyloxy, toluenesulfonyloxy, azido, or -O-CO-($C_4$-$C_7$ alkyl).

The compounds of the present invention are derivatives of benzo[b]thiophene which are named and numbered according to the RING INDEX as follows.

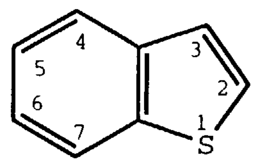

The most preferred compounds employed in the methods of this invention are those compounds of Formula I wherein

b) at least one of $R^a$ and $R^b$ is methylsulfonyl;
c) $R^1$ is hydrogen, hydroxy, or $C_1$-$C_3$ alkoxy;
d) n is 1 to 4; and
e) $R^3$ is piperidinyl, hexamethyleneiminyl,

pyrrolidinyl, or -$NR^4R^5$, where $R^4$ and $R^5$ are $C_1$-$C_4$ alkyl; and the pharmaceutically acceptable acid addition salts and solvates.

The compounds of the present invention can be prepared by a variety of procedures well known to those of ordinary skill in the art. The particular order of steps required to produce the compounds of Formula I is dependent upon the particular compound being synthesized, the starting compound, and the relative lability of the substituted moieties.

The compounds used in the methods of this invention form pharmaceutically acceptable acid and base addition salts with a wide variety of organic and inorganic acids and bases and include the physiologically acceptable salts which are often used in pharmaceutical chemistry. Such salts are also part of this invention. Typical inorganic acids used to form such salts include hydrochloric, hydrobromic, hydroiodic, nitric, sulfuric, phosphoric, hypophosphoric and the like. Salts derived from organic acids, such as aliphatic mono and dicarboxylic acids, phenyl substituted alkanoic acids, hydroxyalkanoic and hydroxyalkandioic acids, aromatic acids, aliphatic and aromatic sulfonic acids, may also be used. Such pharmaceutically acceptable salts thus include acetate, phenylacetate, trifluoroacetate, acrylate, ascorbate, benzoate, chlorobenzoate, dinitrobenzoate, hydroxybenzoate, methoxybenzoate, methylbenzoate, o-acetoxybenzoate, naphthalene-2-benzoate, bromide, isobutyrate, phenylbutyrate, β-hydroxybutyrate, butyne-1,4-dicarboxylate, hexyne-1,4-dicarboxylate, caprate, caprylate, cinnamate, citrate, formate, fumarate, glycollate, heptanoate, hippurate, hydrochloride, lactate, malate, maleate, hydroxymaleate, malonate, mandelate, mesylate, nicotinate, isonicotinate, nitrate, oxalate, phthalate, teraphthalate, phosphate, monohydrogenphosphate, dihydrogenphosphate, metaphosphate, pyrophosphate, propiolate, propionate, phenylpropionate, salicylate, sebacate, succinate, suberate, sulfate, bisulfate, pyrosulfate, sulfite, bisulfite, sulfonate, benzenesulfonate, p-bromobenzenesulfonate, chlorobenzenesulfonate, ethanesulfonate, 2-hydroxyethanesulfonate, methanesulfonate, naphthalene-1-sulfonate, naphthalene-2-sulfonate, p-toluenesulfonate, xylenesulfonate, tartarate, and the like. A preferable salt is the hydrochloride salt.

The pharmaceutically acceptable acid addition salts are typically formed by reacting a compound of Formula I with an equimolar or excess amount of acid. The reactants are generally combined in a mutual solvent such as diethyl ether

or benzene. The salt normally precipitates out of solution within about one hour to 10 days and can be isolated by filtration or the solvent can be stripped off by conventional means.

Bases commonly used for formation of salts include ammonium hydroxide and alkali and alkaline earth metal hydroxides and carbonates, as well as aliphatic and aromatic amines, aliphatic diamines and hydroxy alkylamines. Bases especially useful in the preparation of addition salts include ammonium hydroxide, potassium carbonate, calcium hydroxide, methylamine, diethylamine, ethylene diamine, cyclohexylamine and ethanolamine.

The pharmaceutically acceptable salts frequently have enhanced solubility characteristics compared to the compound from which they are derived, and thus are often more amenable to formulation as liquids or emulsions.

Many of the compounds of the present invention are prepared as described in European Patent Application No. 96302169.6, filed March 28, 1996. That application concerns processes for preparing 2-substituted benzo[b]thiophene compounds by means of forming a 2-position boronic acid derivative of a benzo[b]thiophene and coupling that with an aryl halide or preparing a 2-halo benzo[b]thiophene and coupling that with a boronic acid derivative of a substituted phenyl group. In the above-captioned application there are two routes employed to prepare the desired compounds. These two routes are depicted infra.

**Scheme I**

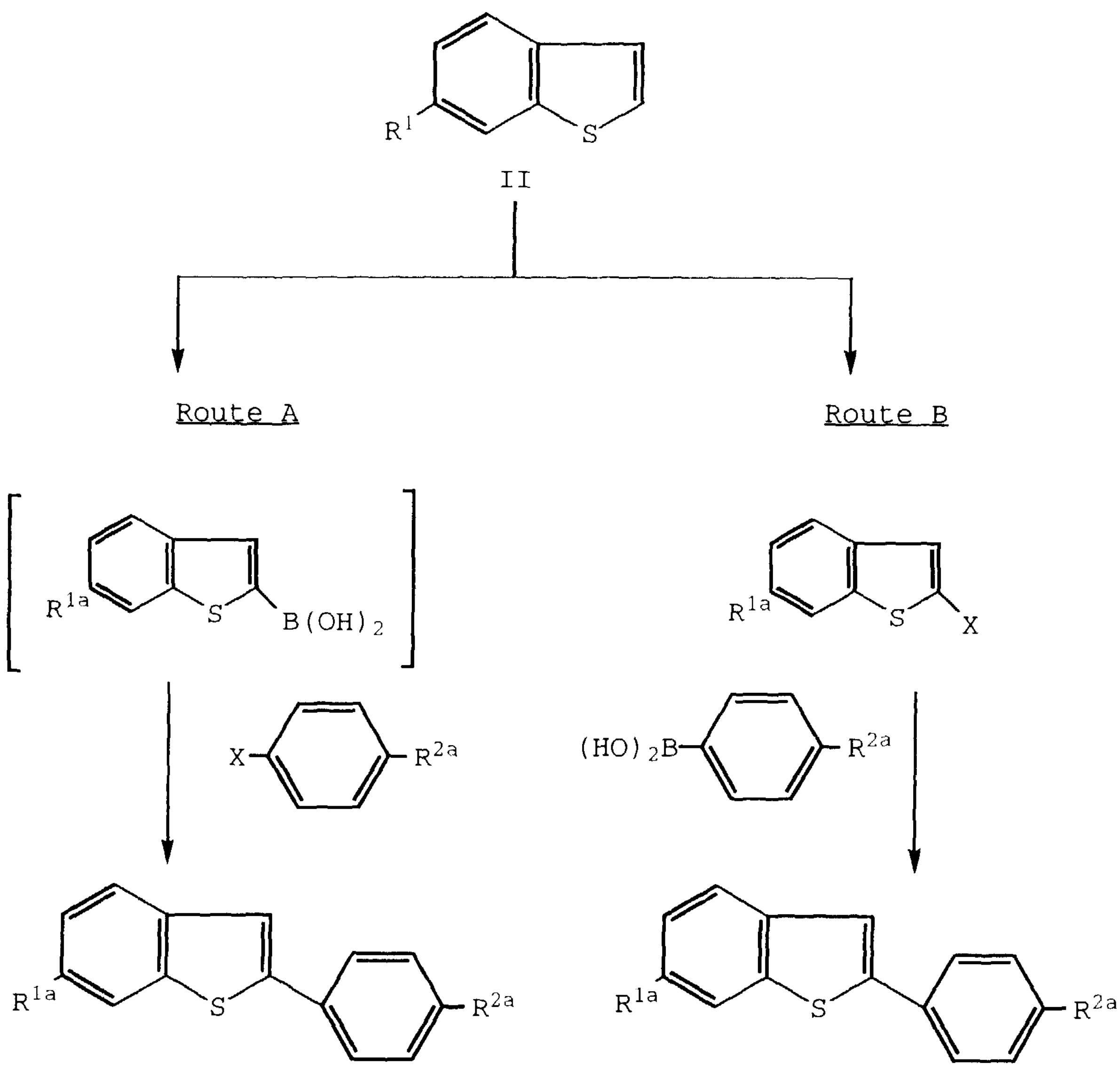


wherein:

X is halo or triflate and $R^{1a}$ and $R^{2a}$ are independently hydrogen, hydroxy, or a hydroxy-protecting group.

In the first step of Route A of Scheme I, a 2-position arylboronic acid is formed using standard procedures. Generally, the 2-unsubstituted benzo[b]thiophene is treated with a slight excess of an n-alkyllithium in hexanes, in an appropriate solvent and, frequently, under an inert atmosphere such as nitrogen, followed by the slow or dropwise addition of an appropriate trialkylborane.

Appropriate solvents include an inert solvent or mixture of solvents such as, for example, diethyl ether, dioxane and tetrahydrofuran (THF). Of these, THF, particularly anhydrous THF, is preferred.

The preferred trialkylborate used in the present reaction is triisopropyl borate.

The product of this reaction, a 2-position arylboronic acid, is then reacted with an aryl compound, via standard Suzuki coupling procedures to provide compounds of formula I. The aryl halides are derived from commercially available compounds via procedures well known to one of ordinary skill in the art [see, e g., ADVANCED ORGANIC CHEMISTRY: REACTIONS, MECHANISMS, AND STRUCTURE, 4th Edition (J. March, ed., John Wiley and Sons, Inc., 1992); and A. Suzuki, Pure and Applied Chemistry, 6:213-222 (1994)].

In the present coupling reaction, a slight excess of the aryl halide is reacted with each equivalent in the presence of a palladium catalyst and an appropriate base in an inert solvent such as toluene.

Although various palladium catalysts drive this coupling reaction, the catalyst selected usually is reaction specific. Thus, the use of tetrakis triphenylphosphine palladium in the present reaction is highly preferred.

Likewise, various bases may be used in the present coupling reaction. However, it is preferred to use an alkali metal carbonate, particularly 2 N sodium carbonate.

The temperature employed in this step should be sufficient to effect completion of the coupling reaction. Typically, heating the reaction mixture to reflux for a period from about 2 to about 4 hours is adequate and preferred.

In Route B of Scheme I, the first step involves the 2-position bromination, iodination, or forming a triflate leaving group of a 2-unsubstituted benzo [b] thiophene using standard procedures. Generally, when brominating or iodinating, the benzo[b] is reacted with a slight excess of n-butyllithium in hexane, in an appropriate solvent and, frequently under an inert atmosphere such as nitrogen, followed by the dropwise addition of a slight excess of the desired brominating or iodinating agent in an appropriate solvent. A preferred iodinating agent is iodine, and preferred brominating agents include bromine and N-bromosuccinimide.

Appropriate solvents include an inert solvent or mixture of solvents such as, for example, diethyl ether, dioxane, or THF. THF is preferred and anhydrous THF is especially preferred.

The present reaction is optionally run at a temperature range from about -75° C to about -85° C.

The product of the above reaction, a halo arene, is then coupled with an arylboronic acid compound to provide compounds of Formula I. The preferred reaction conditions for the coupling reaction are as described for the coupling reaction involving the compounds in Route A of Scheme I above.

The processes shown in Scheme I and herein described may be carried out in separate steps in which the reaction product from each step is purified and characterized, or the process shown in Route A and the process shown in Route B is carried out *in situ.* Thus, the present processes, preferably, are each carried out in a single vessel.

Examples

The following experiments illustrate the preparation of the benzothiophenes employed in the present invention. The terms "NMR", "IR" or "MS" following a synthesis protocol indicates that the nuclear magnetic resonance spectrum, infrared spectrum, or the mass spectrometry was performed and was consistent with the title product.

Preparation 1

Preparation of N,N-dimethyl α-hydroxy-α-(4-methoxyphenyl)thioacetamide

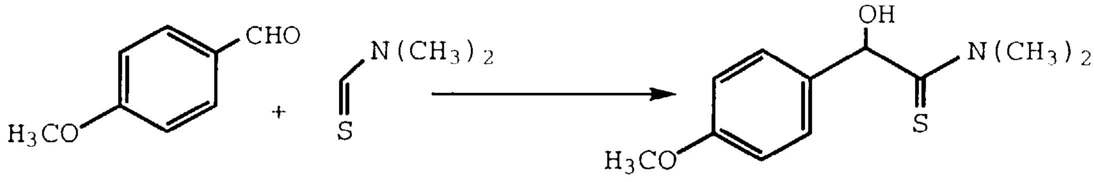

A sample of diisopropylamino (5.66 ml, 40.4 mmol) was dissolved in 27 ml of tetrahydrofuran in a dry 100 ml round bottom flask equipped wtih a magnetic stir bar and septum under a nitrogen atmosphere. The solution mixture was then chilled to 0°C in an ice bath, followed by the addition of n-butyl lithium (24.9 ml, 40.4 mmol, 1.62 M in hexanes). After fifteen minutes of additional mixing, the solution mixture was chilled to -78°C in an acetone/dry ice, followed by

the addition of a 10 ml tetrahydrofuran solution of a mixture of anisaldehyde (5.00 g, 36.7 mmol) and N,N-dimethylthioformamide (3.60 g, 40.4 mmol). A translucent, yellow, heterogeneous mixture forms, which is maintained at -78°C for 2.5 hours, followed by warming the reaction mixture to 0°C in an ice bath.

The reaction mixture was then treated with 20 ml of aqueous saturated ammonium chloride, then diluted with 40 ml of diethyl ether. The aqueous fraction was extracted wtih 20 ml of diethyl ether. The organic fractions were combined, dried over sodium sulfate, and then filtered. The solvents were removed in vacuo to yield 8.87 grams of a solid/oil mixture.

This mixture was then slurried with 10 ml of diethyl ether, and then chilled to 0°C in an ice bath. The mixture was then suction-filtered cold, followed by washing of the filter cake with ice-cold diethyl ether (2 x 5 ml). The wet solid was then further dried under vacuum to yield an off-white powder (3.90 grams). An additional crop of crystals were obtained from the combined mother liquor and ethereal washings (0.84 grams total). The total yield was 57%. If desired the title product could be further purified by recrystallization from ethanol, although this procedure is not necessary.

Preparation 2

Preparation of 2-dimethylamino-6-methoxybenzo[b]thiophene

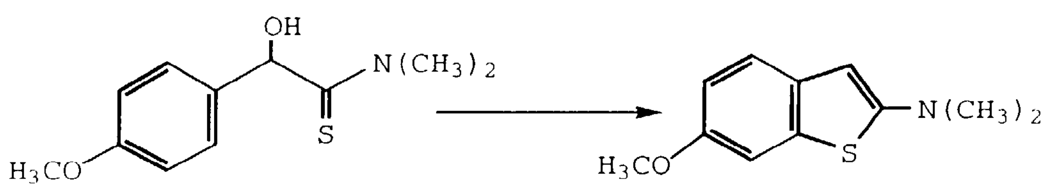

A sample of N,N-dimethyl 2-hydroxy-2-(4-methoxyphenyl)thioacetamide (40.0 g, 177 mmol) was dissolved in 1480 ml of methylene chloride in a 3000 ml 3-neck flask equipped with a mechanical stirrer and digital temperature monitor apparatus. A sample of methanesulfonic acid (57.0 ml, 888 mmol) was then added slowly with vigorous stirring (18.9 → 24.6°C) . The reaction was then allowed to proceed for about two hours, at which point consumption of starting material was confirmed by thin layer chromatography analysis. The deep red reaction mixture was then treated with 300 ml of aqueous sodium carbonate, followed by 100 ml of water with vigorous stirring (21.7 → 28.0°C). The layers were separated and the organic phase was dried with solid sodium chloride (~5 g), decanted, and then concentrated under reduced pressure to yield a solid (51.0 g) (solvent entrained). This solid was the recrystallized from 200 ml of ethanol which yielded a yellow solid which was dried at 50°C overnight in vacuo. Yield: 29.2 grams (79%) . mp 75-76°C.

Preparation 3

Preparation of 4-[2-(piperidin-1-yl)ethoxy]benzoyl chloride hydrochloride

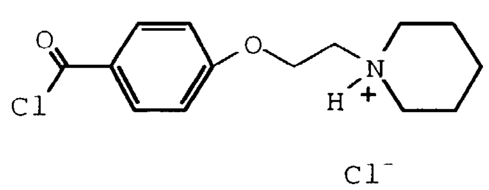

The title compound was prepared essentially as described in PCT Patent Application Number PCT/US95/11872, filed 19 September 1995.

A. Ethyl 4-[2-(piperidin-1-yl)ethoxyl]benzoate

A mixture of ethyl 4-hydroxybenzoate (8.31 g), 1-(2-chloro-ethyl)piperidine monohydrochloride (10.13 g), potassium carbonate (16.59 g), and methyl ethyl ketone (60 ml) was heated to 80°C. After one hour, the mixture was cooled to about 55°C and treated with additional 1-(2-chloroethyl)piperidine mono-hydrochloride (0.92 g). The resulting mixture was heated to 80°C. The reaction was monitored by thin layer chromatography (TLC), using silica-gel plates and ethyl acetate/acetonitrile/ triethylamine (10:6:1, v/v). Additional portions of 1-(2-chloroethyl)piperidine hydrochloride were added until the starting 4-hydroxybenzoate ester was consumed. Upon complete reaction, the reaction mixture was

treated with water (60 ml) and allowed to cool to room temperature. The aqueous layer was discarded and the organic layer concentrated *in vacuo* at 40°C and 40 mm Hg. The resulting oil was used in the next step without further purification.

B. 4-[2-(Piperidin-1-yl)ethoxy]benzoic acid hydrochloride

A solution of the compound prepared as described supra (about 13.87 g) in methanol (30 ml) was treated with 5 N sodium hydroxide (15 ml), and heated to 40°C. After 4 1/2 hours, water (40 ml) was added. The resulting mixture was cooled to 5-10°C, and concentrated hydrochloric acid (18 ml) was added slowly. The title compound crystallized during acidification. This crystalline product was collected by filtration, and dried in vacuo at 40-50°C to give 83% yield of the title compound. Melting point 270-271°C.

C. 4-[2-(Piperidin-1-yl)ethoxy]benzoyl chloride hydrochloride

A solution of the compound prepared as described above (30.01 g) and dimethylformamide (2 ml) in methylene chloride (500 ml) was treated with oxalyl chloride (10.5 ml) over a 30-35 minute period. After stirring for about 18 hours, the reaction was assayed for completion by HPLC analysis. Additional oxalyl chloride may be added to the reaction if the starting carboxylic acid is present. Upon completion, the reaction solution was evaporated to dryness in vacuo. The residue was dissolved in methylene chloride (200 ml), and the resulting solution evaporated to dryness. This dissolution/evaporation procedure was repeated to give the title compound as a solid. The title compound may be stored as a solid or as a 0.2 M solution in methylene chloride (500 ml).

<u>Preparation 4</u>

Preparation of 2-dimethylamino-6-methoxy-3-[4-[2-(piperidin-1-yl)ethoxy]benzoyl]benzo[b]thiophene

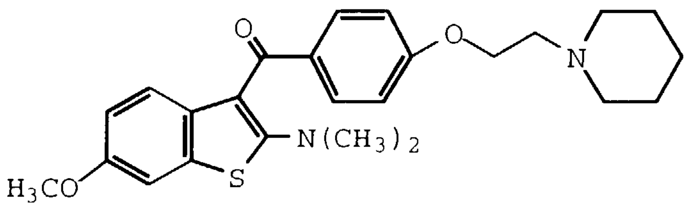

A sample of 2-dimethylamino-6-methoxybenzo[b]thiophene (10.3 g, 49.8 mmol) and 4-[2-(piperidin-1-yl)ethoxy] benzoyl chloride hydrochloride (15.9 g, 52.3 mmol) were partially dissolved in 100 ml of chlorobenzene in a 250 ml round bottom flask equipped with a reflux condensor, magnetic stir bar, and septum, under a dry nitrogen atmosphere. The resulting mixture was heated to 100-105°C in an oil bath and maintained at that temperature for about nine hours. The mixture was then permitted to cool to room temperature over about one hour, resulting in solidification.

The solid was then broken up and treated with aqueous saturated sodium carbonate (60 ml), followed by water (30 ml), then methylene chloride, then 50% aqueous sodium hydroxide (10 ml). After stirring for a short period, the mixture was partitioned between 300 ml of methylene chloride and 100 ml of water. The layers were separated and the organic fraction was washed with 50% saturated sodium carbonate (40 ml). The organic fraction was then dried over solid sodium chloride (~5 grams) and then decanted. The solvents were removed in vacuo to yield a thick dark oil (24.6 g). The desired title product was further purified by chromatography to yield 19.8 grams (91%).

Example 1

Preparation of 2-(4-dimethylaminophenyl)-6-methoxy-3-[4-[2-(piperidin-1-yl)ethoxy]benzoyl]benzo[b]thiophene

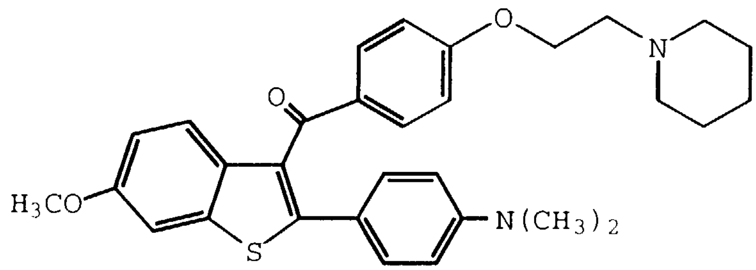

To 2-dimethylamino-6-methoxy-3-[4-[2-(piperidin-1-yl)ethoxy]benzoyl]benzo[b]thiophene (1.5 g, 3.4 mmol) stirring in 10 ml of dry tetrahydrofuran at 0°C under a nitrogen atmosphere was added over a ten minute period 12.5 ml of a 0.70 M tetrahydrofuran solution of 4-N,N-dimethylanilinemagnesium bromide (8.8 mmol). The reaction mixture was stirred under nitrogen and allowed to warm gradually to room temperature. After 24 hours the reaction was quenched with a saturated aqueous solution of ammonium chloride, then washed twice with methylene chloride.

The combined organic layers were dried over magnesium sulfate, filtered, then concentrated in vacuo. The crude product was purified by column chromatography (silica gel, 10% methanol in 1:1 hexanes:ethyl acetate) to provide 1.1 grams (63%) of the title compound as an orange-brown solid.
$^1$H NMR (300 MHz, $CDCl_3$) δ 1.62 (m, 2H), 1.83 (m, 4H), 2.78 (m, 4H), 3.03 (t, 2H), 3.05 (s, 6H), 4.00 (s, 3H), 4.33 (t, 2H), 6.67 (d, 2H), 6.89 (d, 2H), 7.07 (dd, 1H), 7.39-7.43 (m, 3H), 7.91 (d, 2H).

Example 2

Preparation of 2-(4-dimethylaminophenyl)-6-hydroxy-3-[4-[2-(piperidin-1-yl)ethoxy]benzoyl]benzo[b]thiophene

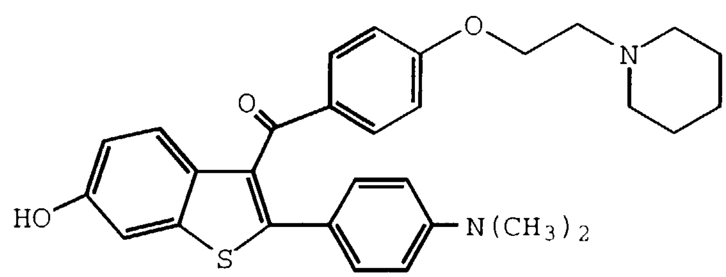

To 2-(4-dimethylaminophenyl)-6-methoxy-3-[4-[2-(piperidin-1-yl)ethoxy]benzoyl]benzo[b]thiophene (1.1 g, 2.1 mmol), as prepared in Example 1 and ethanediol (1.0 ml, 13.5 mmol), stirring at room temperature under nitrogen in 30 ml anhydrous methylene chloride, was added aluminum chloride (2.85 g, 21.4 mmol). The reaction mixture was stirred at room temperature under nitrogen for 2.5 hours. The reaction was quenched with a saturated aqueous solution of sodium bicarbonate, then diluted with ethyl acetate.

The organic fraction was washed three times with a saturated aqueous solution of sodium bicarbonate. The aqueous layer was washed twice with ethyl acetate. The combined organic layers were dried over magnesium sulfate, filtered, then concentrated in vacuo. The crude product was purified by column chromatography (silica gel, 1% to 10% gradient of methanol in 1:1 ethyl acetate:methylene chloride) to provide 0.60 grams (57%) of the title product as a green foam.
$^1$H NMR (300 MHz, $CDCl_3$) δ 1.44 (m, 2H), 1.64 (m, 4H), 2.56 (m, 4H), 2.78 (t, J=5.52 Hz, 2H), 2.87 (s, 6H), 4.07 (t, J=5.52 Hz, 2H), 6.51 (d, J=8.83 Hz, 2H), 6.62 (d, J=8.83 Hz, 2H), 6.74 (dd, J=2.21, 8.82 Hz, 1H), 7.15 (d, J=1.84 Hz, 1H), 7.23 (d, J=8.82 Hz, 2H0, 7.33 (d, J=8.82 Hz, 1H), 7.72 (d, J=8.83 Hz, 2H).
$^{13}$C NMR (75 MHz, $CDCl_3$) δ 23.8, 25.2, 40.2, 54.9, 57.6, 65.2, 107.5, 112.0, 114.1, 115.1, 121.3, 123.6, 129.8, 130.6, 132.4, 133.6, 139.8, 143.2, 150.2, 154.4, 162.6, 194.1
IR ($CHCl_3$) 1641, 1600 cm$^{-1}$.

| High Resolution FDMS: | |
|---|---|
| Theory: | 501.2212 ($MH^+$) |
| Found: | 501.2213 |

| Analysis for $C_{30}H_{32}N_2O_3S$: | | | |
|---|---|---|---|
| Theory: | C, 49.67; | H, 6.61; | N, 5.40. |
| Found: | C, 70.14; | H, 6.40; | N, 5.40. |

Preparation 5

Preparation of 4-[N,N-(dimethanesulfonamido)]-bromobenzene.

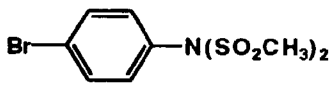

To a solution of 4-bromoanaline (17.20 ml, 0.10 mol) in 100 ml of anhydrous methylene chloride at 0°C was added N,N-diethylisopropyl amine (39.0 ml, 0.22 mol). To this solution was added methanesulfonylchloride (17.20 ml, 0.22 mol) dropwise as a solution in 30 ml of anhydrous methylene chloride. The resulting mixture was allowed to gradually warm to room temperature and stirred for 1 h. The reaction mixture was then distributed between $H_2O$/EtOAc. A precipitate (amine hydrochloride) formed in the organic layer that was removed by filtration. The organic was dried ($Na_2SO_4$) and concentrated *in vacuo* to a brown solid. Crystallization from EtOAc provided 23.54 g (74%) of 4-[N,N-(dimethanesulfonamido)]bromobenzene as a white solid.
mp 222-225 °C. FDMS 327.
$^1$H NMR (DMSO-$d_6$) δ 7.66 (d, J = 8.6 Hz, 2H), 7.45 (d, J= 8.6 Hz, 2H), 3.50 (s, 3H).

| Analysis for $C_8H_{10}BrNO_4S_2$: | | | |
|---|---|---|---|
| Theory: | C, 29.28; | H, 3.07; | N, 4.27. |
| Found: | C, 29.25; | H, 3.01; | N, 4.23. |

Preparation 6

Prepared by the same procedure in 90% yield from 4-bromoanaline and triflouromethanesulfonic anhydride was 4-(N, N-(di-triflouromethanesulfonamido))-bromobenzene.

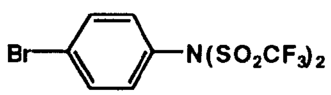

mp 59-61 °C. FDMS 435, 437
$^1$H NMR ($CDCl_3$) δ 7.63 (d, J = 8.5 Hz, 2H), 7.28 (d, J = 8.5 Hz, 2H).

| Analysis for $C_8H_4BrF_6NO_4S_2$: | | | |
|---|---|---|---|
| Theory: | C, 22.03; | H, 0.92; | N, 3.21. |
| Found: | C, 22.03; | H, 0.90; | N, 3.15. |

Preparation 7

Preparation of [6-methoxy]-2-[4-(N,N-dimethanesulfonamido)phenyl]benzo[b]thiophene.

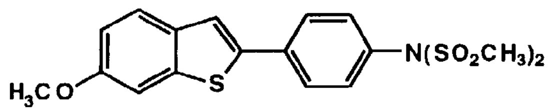

To a solution 6-methoxybenzo[b]thiophen-2-boronic acid (1.98 g, 95.0 mmol) in 100 ml of toluene was added 4-(N,N-(dimethanesulfonamido))-bromobenzene (3.12 g, 95.0 mmol), tetrakistiphenylphosphinepalladium (0.33 g, 0.30 mmol), 10.4 ml of 2 N sodium carbonate solution, and 10 ml of absolute ethanol. The resulting mixture was heated to reflux for 4 hours.

Upon cooling a solid formed. The mixture was diluted with ethyl acetate and water. The aqueous layer was removed, and the organic layer was concentrated to a solid. The solid was slurried in ethyl acetate and then collected by filtration to provide 2.78 g (71%) of [6-methoxy] -2- [4- (N,N-dimethanesulfonamido)phenyl]benzo[b]thiophene as an amber solid. mp 250 °C (dec). FDMS 411.

$^1$H NMR (DMSO-$d_6$) δ 7.85 (s, 1H), 7.78 (d, J = 8.4 Hz, 2H), 7.72 (d, J = 8.5 Hz, 1H), 7.56 (d, J = 8.5 Hz, 1H), 7.55 (d, J = 8.4 Hz, 2H), 6.99 (dd, J = 8.4, 2.2 Hz, 1H), 3.80 (s, 3H), 3.53 (s, 6H).

| Analysis for $C_{17}H_{17}NO_5S_3$: | | | |
|---|---|---|---|
| Theory: | C, 49.62; | H, 4.16; | N, 3.40. |
| Found: | C, 49.90; | H, 4.07; | N, 3.10. |

Preparation 8

By the same procedure, 4-(N,N-(di-triflouromethanesulfonamido))-bromobenzene was reacted with 6-methoxybenzo[b]thiophene-2-boronic acid to give [6-methoxy]-2-[4-(N-triflouromethanesulfonamido)phenyl]-benzo[b]thiophene in 55% yield as an amber solid.

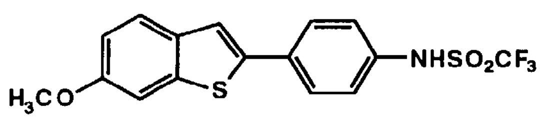

mp 260 °C (dec) . FDMS 387

$^1$H NMR (DMSO-$d_6$) δ 7.61 (d, J = 8.7 Hz, 1H), 7.46 (s, 1H), 7.45 (d, J = 2.2 Hz, 1H), 7.38 (d, J = 8.5 Hz, 2H), 6.98 (d, J = 8.5 Hz, 2H), 6.93 (dd, J = 8.7, 2.2 Hz, 1H), 3.78 (s, 3H).

Example 3

Preparation of 2-[4-(N,N-dimethylsulfonylamino)phenyl]-6-methoxy-3-[4-[2-(piperidin-1-yl)ethoxy]benzoyl]benzo[b]thiophene

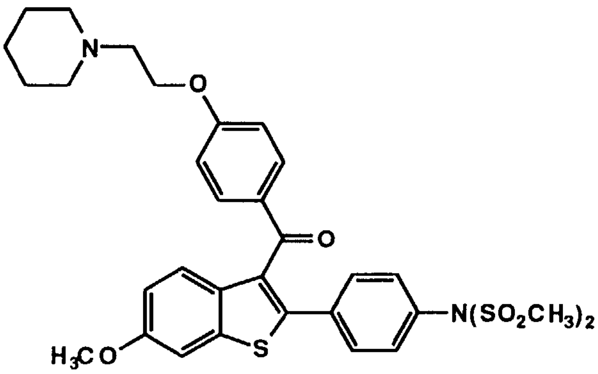

To a solution of [6-methoxy]-2-[4-(N,N-dimethanesulfonamido)phenyl]benzo[b]thiophene (1.00 g, 2.43 mmol) and 4-[2-(1-piperidinyl)ethoxy]-benzoyl chloride hydrochloride (0.815 g, 2.68 mmol) in 40 ml of anhydrous methylene chloride under nitrogen at -5°C was added $AlCl_3$ (5.20 g, 39.0 mmol). The resulting mixture was allowed to warm to room temperature and stirred for 18 hours. The mixture was then poured into a saturated sodium bicarbonate solution. The aqueous was then extracted several times with ethyl acetate. The organic was dried over sodium sulfate and concentrated in vacuo to a foam. Chromatography ($SiO_2$, 1-3% MeOH/$CHCl_3$) provided 1.37 g (91%) of the title compound as a yellow oil.
FDMS 643.

Example 4

Prepared in an analogous manner and isolated in 48% yield as a yellow solid was 2-[4-(trifluoromethylsulfonamido)phenyl]-6-methoxy-3-[4-[2-(piperidin-1-yl)ethoxy]benzoyl]benzo[b]thiophene.

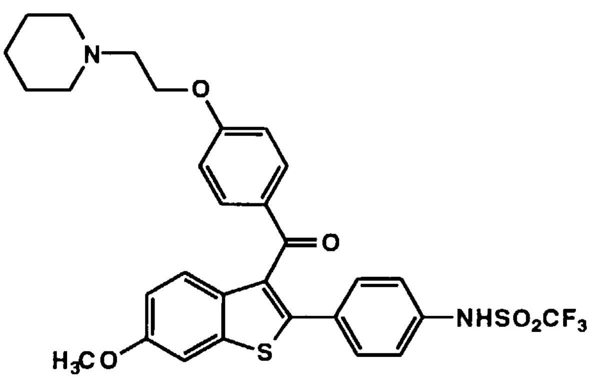

mp 140-145 °C. FDMS 619.
$^1H$ NMR ($CDCl_3$) δ 8.05 (d, J = 8.9 Hz, 2H), 7.54 (d, J = 8.7 Hz, 2H), 7.32 (d, J = 2.2 Hz, 1H), 7.04 (dd, J = 8.9, 2.2 Hz, 1H), 6.90 (d, J = 8.8 Hz, 2H), 6.87 (d, J = 8.8 Hz, 2H), 6.54 (d, J = 8.7 Hz, 2H), 4.35 (m, 2H), 3.32-3.19 (m, 6H), 1.99-1.94 (m, 4H), 1.39-1.24 (m, 2H).

Analysis for $C_{30}H_{29}F_3N_2O_5S_2$ • 1.5 $H_2O$:

| | | | |
|---|---|---|---|
| Theory: | C, 55.80; | H, 4.99; | N, 4.34. |
| Found: | C, 55.90; | H, 4.82; | N, 4.22. |

Example 5

Preparation of 2-[4-(N,N-dimethylsulfonylamino)phenyl]-6-hydroxy-3-[4-[2-(piperidin-1-yl)ethoxy]benzoyl]benzo[b]thiophene

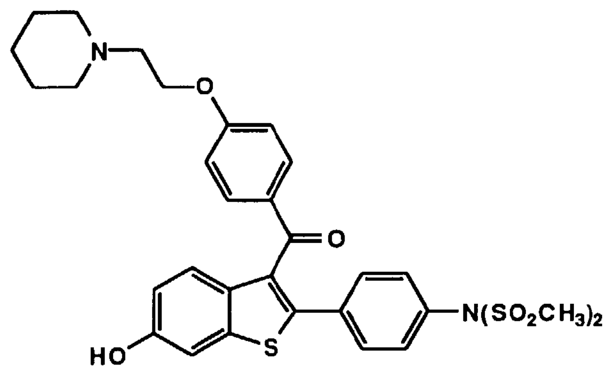

To a solution of 2-[4-(N,N-dimethylsulfonylamino)phenyl]-6-methoxy-3-[4-[2-(piperidin-1-yl)ethoxy]benzoyl]benzo[b]thiophene (0.65 g, 1.00 mmol) in 15 ml of anhydrous methylene chloride under nitrogen at 0°C was added $AlCl_3$ (0.80 g, 6.00 mmol). To this mixture was added EtSH (0.45 ml, 36.0 mmol). The resulting solution was warmed to room temperature and stirred for 3 h. The reaction was quenched by pouring into sat. $NaHCO_3$ solution. The aqueous solution was extracted several times with EtOAc. The organic was then dried ($Na_2SO4$) and concentrated to a yellow oil. Chromatography ($SiO_2$, 5% MeOH/$CHCl_3$) provided 444 mg (71%) of the title compound as a yellow solid. mp 136-140°C. FDMS 629.
$^1$H NMR ($CDCl_3$) δ 7.57 (d, J = 8.7 Hz, 2H), 7.48 (d, J = 8.8 Hz, 1H), 7.39 (d, J = 8.3 Hz, 2H), 7.16 (d, J = 8.3 Hz, 2H), 7.15 (d, J = 2.2 Hz, 1H), 6.81 (dd, J = 8.8, 2.2 Hz, 1H), 6.57 (d, J = 8.7 Hz, 2H), 4.08 (bt, J = 6.0 Hz, 2H), 3.33 (s, 6H), 2.79 (bt, J = 6.0 Hz, 2H), 2.60 (m, 4H), 1.67-1.48 (m, 4H), 1.28-1.23 (m, 2H).

| Analysis for $C_{30}H_{32}N_2O_7S_3$ : | | | |
|---|---|---|---|
| Theory: | C, 57.31; | H, 5.13; | N, 4.46. |
| Found: | C, 57.46; | H, 5.23; | N, 4.42. |

Example 6

Prepared by the same procedure in 63% yield was 2-[4-(trifluoromethylsulfonamido)phenyl]-6-hydroxy-3-[4-[2-(piperidin-1-yl)ethoxy]benzoyl]benzo[b]thiophene.

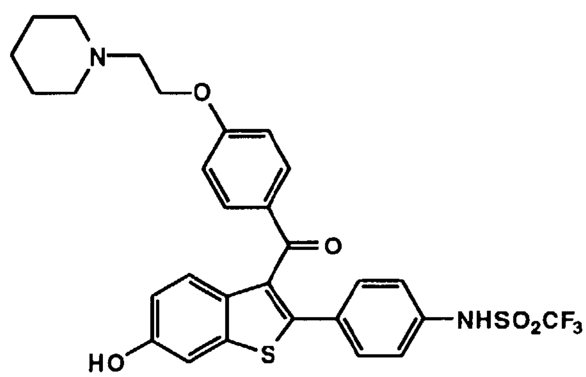

mp 180°C (dec). FDMS 605
$^1H$ NMR (DMSO-$d_6$) δ 9.73 (s, 1H), 7.66 (d, J = 8.7 Hz, 2H), 7.30 (d, J = 2.2 Hz, 1H), 7.25 (d, J = 8.7 Hz, 1H), 7.01 (d, J = 8.50 Hz, 2H), 6.93 (d, J = 8.50 Hz, 2H), 6.83 (dd, J = 8.7, 2.2 Hz, 1H), 6.80 (d, J = 8.7 Hz, 2H), 4.31 (m, 2H), 3.45-2.96 (m, 6H), 1.76-1.20 (m, 6H).

| Analysis for $C_{29}H_{27}F_3N_2O_5S_2$ : | | | |
|---|---|---|---|
| Theory: | C, 57.61; | H, 4.50; | N, 4.63. |
| Found: | C, 57.33; | H, 4.64; | N, 4.37. |

Example 7

Preparation of 2-[4-(methylsulfonamido)phenyl]-6-hydroxy-3-[4-[2-(piperidin-1-yl)ethoxy]benzoyl]benzo[b]thiophene.

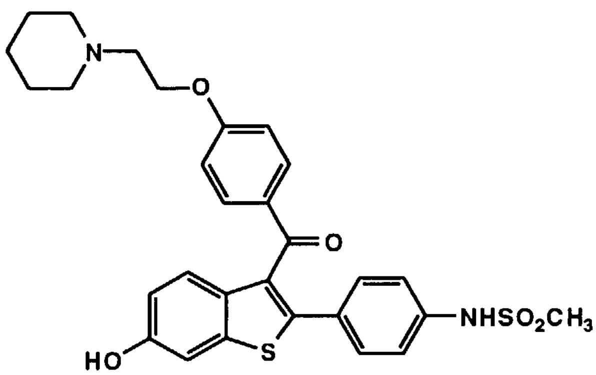

2-[4-(N,N-dimethylsulfonylamino)phenyl]-6-methoxy-3-[4-[2-(piperidin-1-yl)ethoxy]benzoyl]benzo[b]thiophene (101 mg, 0.16 mmol) was dissolved in 30 ml of a 2:1 mixture of tetrahydrofuran and 1 N sodium hydroxide. The resulting mixture was heated to reflux for 6 hours. Upon cooling, the tetrahydrofuran was removed in vacuo. The pH of the aqueous layer was adjusted to 7.0 using 1 N hydrochloric acid. The aqueous was then extracted several times with ethyl acetate. The organic was dried over sodium sulfate and concentrated in vacuo to a yellow solid that was triturated from diethyl ether. Filtration provided 64 mg (70%) of the title compound. mp 130 °C (dec). FDMS 551.

| Analysis for $C_{29}H_{30}N_2O_5S_2 \cdot H_2O$ : | | | |
|---|---|---|---|
| Theory: | C, 61.25; | H, 5.67; | N, 4.93. |
| Found: | C, 60.94; | H, 5.70; | N, 4.64. |

By substantially following the procedures described above one skilled in the art can prepare the other compounds of Formula I.

The current invention concerns the discovery that a select group of benzo[b]thiophenes, those of Formula I, are useful for treating resistant neoplasms. The methods of treatment provided by this invention are practiced by administering to a human or other mammal in need thereof a multidrug resistance reversing amount of a compound of Formula I or a pharmaceutically acceptable salt or solvate thereof, that is effective to make the neoplasms less resistant to chemotherapy. In making the neoplasm less resistant, the compounds of the invention may be used on neoplasms having intrinsic and/or acquired resistance. Such neoplasms include those which have a pathway for resistance which includes the protein p190. Resistance to drugs such as epipodophyllotoxins and anthracyclines are linked to p190. The treatment of the resistant and susceptible neoplasm will result in a reversal or inhibition of resistance, or in other words, will cause the neoplasm to be more sensitive to the appropriate chemotherapy such as treatment with vinblastine, vincristine, vindesine, navelbine, daunorubicin, doxorubicin, mitroxantrone, etoposide, teniposide, mitomycin C, actinomycin D, taxol, topotecan, mithramycin, colchicine, puromycin, podophyllotoxin, ethidium bromide, emetine, gramicidin D, and valinomycin.

The compounds of the present invention may be used for many resistant neoplasms, including colon cancer, mesothelioma, melanoma, prostate cancer, ovarian cancer, non-small cell lung cancer, small-cell lung cancer, bladder cancer, endometrial cancer, leukemia, renal cancer, liver cancer, neurological tumors, testicular cancer, breast cancer, and large cell lymphoma. More particular types of cancer are Hodgkin's disease, Karposi's sarcoma, and acute granulocytic leukemia.

The biological activity of the compounds of the present invention was evaluated employing an initial screening

assay which rapidly and accurately measured the activity of the tested compound in reversing the resistance present in a multidrug resistant tumor. Assays useful for evaluating this reversing capability are well known in the art. See, e. g., T. McGrath, et al., Biochemical Pharmacology, 38 : 3611, (1989) ; D. Marquardt and M.S. Center, Cancer Research, 52:3157, (1992); and D. Marquardt, et al., Cancer Research, 50 : 1426, (1990).

Assay for Reversal of p190-Mediated Doxorubicin Resistance

HL60/ADR is a continuous cell line which was selected for ADRIAMYCIN™ resistance by culturing HL60, a human acute myeloblastic leukemia cell line, in increasing concentrations of ADRIAMYCIN™ until a highly resistant variant was attained.

HL60/ADR cells were grown in RPMI 1640 (Gibco) containing 10% fetal bovine serum (FBS) and 250 µg/ml GENTAMICIN™ (Sigma). Cells were harvested; washed twice with assay medium (same as culture media); counted; and diluted to 2 x $10^5$ cells/ml in assay medium. Fifty microliters of cells were aliquoted into wells of a 96 well tissue culture plate. One column of each 96 well plate served as a negative control and received assay medium containing no cells.

Test compounds and references compounds were dissolved in dimethyl sulfoxide (DMSO) at a concentration of 5 mM. Samples were diluted to 20 µM in assay medium and 25 µl of each test compound was added to 6 wells. Assay standards were run in quadruplicate. Twenty-five microliters of 0.4% DMSO was added to four wells as a solvent control. Assay media was added to all wells to achieve a final volume of 100 µl per well.

The plates were incubated at 37°C for 72 hours in a humidified incubator with a 5% carbon dioxide atmosphere. Cell viability and vitality was measured by oxidation of a tetrazolium salt using standard conditions. The plates were incubated for 3 hours at 37° C. Absorbance was determined at 490 nm using a microtitre plate reader.

The ability of a test compound to reverse the resistance of HL60/ADR cells to an oncolytic was determined by comparison of the absorbance of the wells containing a test compound in addition to the oncolytic (such as ADRIAMYCIN™) with the absorbance of wells containing the oncolytic without a test compound. Controls were used to eliminate background and to ensure the results were not artifactual. The results of the assay are expressed as percent inhibition of cell growth. The oncolytic alone at the tested concentration does not usually inhibit the growth of HL60/ADR cells.

Assay for Reversal of P-Glycoprotein-Mediated Doxorubicin Resistance

The human cell leukemia cell lines CCRF-CEM and the multidrug resistant CEM/VLB100 [selected against 100 ng/ml vinblastine sulfate, as described in W.T. Beck, et al., Cancer Research, 39 : 2070-2076 (1979)] were used to determine the ability of the compounds of the present invention to reverse multidrug resistance mediated by the P-glycoprotein. The cells were maintained in SMEM medium supplemented with 10% fetal bovine serum and 2 mM l-glutamine in a humidified incubator with 5% added carbon dioxide. Cell numbers were determined suing a Coulter Counter model ZM™. Cells were subcultured every 3-4 days.

Cell viability was determined using a modified MTT [3-(4,5-dimethylthiazol-2-yl)-2,5-diphenyltetrazolium bromide] dye reduction methods. See, F. Denziot and R. Lang, Journal of Immunological Methods, 89:271-277 (1986). Cells were harvested during the logarithmic growth phase, and seeded in 96-well seroculture plates at 7.5 x $10^3$ cells/well and cultured for 72 hours in the presence of serially diluted oncolytics. The oncolytics employed were vinblastine sulfate, ADRIAMYCIN™, ETOPOSIDE™, and taxol. These compounds were used with and without the compounds of the present invention.

Initial leads were discovered by a single well assay using a fixed concentration of vinblastine sulfate (4 ng/ml) and modulator (5 µM). The cytotoxicity of the modulator of the prsent invention alone was also determined. Modulators were prepared as 2 mM stocks in dimethylsulfoxide and added to the wells to give a final concentration ranging from 5 µM to 0.5 µM. After 72 hours, 20 µl of freshly prepared MTT (5 mg/ml in Dulbecco's phosphate buffered saline, pH 7.5) was added to each well and placed for four hours in a 37°C incubator.

Cells were pelleted and 70 µl of cell pellet was carefully removed from each well. To this cell pellet were added 100 µl of 2-propanol/0.04 N hydrochloric acid to dissolve the blue formazan-stained cells. Cells were resuspended 5-10 times with a multipipettor or until no particulate matter was visible. The plates were then immediately read with a microplate reader at a wavelength of 570 nm and a reference wavelength of 630 nm. The controls were measured in quadruplicate and those containing modulator were measured in duplicate.

The amount of drug, modulator, or drug and modulator that inhibited fifty percent of the growth of the cells ($IC_{50}$) was calculated from semilog dose response curves in the presence and absence of modulators for both the parent and the resistant cell lines. The fold shift was calculated as the $IC_{50}$ for cells treated with oncolytic alone divided by the $IC_{50}$ for cells treated with oncolytic and modulator.

The compounds of Formula I demonstrated a significant effect in reversing the P-190 and P-glycoprotein mediated multiple drug resistances. Many of the compounds showed very significant enhancement of activity in combination with the oncolytic agent as opposed to the oncolytic agent alone.

The compounds of Formula I are usually administered in the form of pharmaceutical compositions. These compounds can be administered by a variety of routes including oral, rectal, transdermal, subcutaneous, intravenous, intramuscular, and intranasal. These compounds are effective as both injectable and oral compositions. Such compositions are prepared in a manner well known in the pharmaceutical art and comprise at least one active compound.

The present invention also includes methods employing pharmaceutical compositions which contain, as the active ingredient, the compounds of Formula I associated with pharmaceutically acceptable carriers. In making the compositions of the present invention the active ingredient is usually mixed with an excipient, diluted by an excipient or enclosed within such a carrier which can be in the form of a capsule, sachet, paper or other container. When the excipient serves as a diluent, it can be a solid, semisolid, or liquid material, which acts as a vehicle, carrier or medium for the active ingredient. Thus, the compositions can be in the form of tablets, pills, powders, lozenges, sachets, cachets, elixirs, suspensions, emulsions, solutions, syrups, aerosols (as a solid or in a liquid medium), ointments containing for example up to 10% by weight of the active compound, soft and hard gelatin capsules, suppositories, sterile injectable solutions, and sterile packaged powders.

In preparing a formulation, it may be necessary to mill the active compound to provide the appropriate particle size prior to combining with the other ingredients. If the active compound is substantially insoluble, it ordinarily is milled to a particle size of less than 200 mesh. If the active compound is substantially water soluble, the particle size is normally adjusted by milling to provide a substantially uniform distribution in the formulation, e.g. about 40 mesh.

Some examples of suitable excipients include lactose, dextrose, sucrose, sorbitol, mannitol, starches, gum acacia, calcium phosphate, alginates, tragacanth, gelatin, calcium silicate, microcrystalline cellulose, polyvinylpyrrolidone, cellulose, water, syrup, and methyl cellulose. The formulations can additionally include: lubricating agents such as talc, magnesium stearate, and mineral oil; wetting agents; emulsifying and suspending agents; preserving agents such as methyl- and propylhydroxybenzoates; sweetening agents; and flavoring agents. The compositions of the invention can be formulated so as to provide quick, sustained or delayed release of the active ingredient after administration to the patient by employing procedures known in the art.

The compositions are preferably formulated in a unit dosage form, each dosage containing from about 5 to about 100 mg, more usually about 10 to about 30 mg, of the active ingredient. The term "unit dosage form" refers to physically discrete units suitable as unitary dosages dosages for human subjects and other mammals, each unit containing a predetermined quantity of active material calculated to produce the desired therapeutic effect, in association with a suitable pharmaceutical excipient.

The active compound is effective over a wide dosage range. For examples, dosages per day normally fall within the range of about 0.5 to about 30 mg/kg of body weight. In the treatment of adult humans, the range of about 1 to about 15 mg/kg/day, in single or divided dose, is especially preferred. However, it will be understood that the amount of the compound actually administered will be determined by a physician, in the light of the relevant circumstances, including the condition to be treated, the chosen route of administration, the actual compound administered, the age, weight, and response of the individual patient, and the severity of the patient's symptoms, and therefore the above dosage ranges are not intended to limit the scope of the invention in any way. In some instances dosage levels below the lower limit of the aforesaid range may be more than adequate, while in other cases still larger doses may be employed without causing any harmful side effect, provided that such larger doses are first divided into several smaller doses for administration throughout the day.

For preparing solid compositions such as tablets the principal active ingredient is mixed with a pharmaceutical excipient to form a solid preformulation composition containing a homogeneous mixture of a compound of the present invention. When referring to these preformulation compositions as homogeneous, it is meant that the active ingredient is dipsersed evenly throughout the composition so that the composition may be readily subdivided into equally effective unit dosage forms such as tablets, pills and capsules. This solid preformulation is then subdivided into unit dosage forms of the type described above containing from 0.1 to about 500 mg of the active ingredient of the present invention.

The tablets or pills of the present invention may be coated or otherwise compounded to provide a dosage form affording the advantage of prolonged action. For example, the tablet or pill can comprise an inner dosage and an outer dosage component, the latter being in the form of an envelope over the former. The two components can be separated by enteric layer which serves to resist disintegration in the stomach and permit the inner component to pass intact into the duodenum or to be delayed in release. A variety of materials can be used for such enteric layers or coatings, such materials including a number of polymeric acids and mixtures of polymeric acids with such materials as shellac, cetyl alcohol, and cellulose acetate.

The liquid forms in which the novel compositions of the present invention may be incorporated for administration orally or by injection include aqueous solutions, suitably flavored syrups, aqueous or oil suspensions, and flavored emulsions with edible oils such as cottonseed oil, sesame oil, coconut oil, or peanut oil, as well as elixirs and similar pharmaceutical vehicles.

Compositions for inhalation or insufflation include solutions and suspensions in pharmaceutically acceptable, aqueous or organic solvents, or mixtures thereof, and powders. The liquid or solid compositions may contain suitable pharmaceutically acceptable excipients as described supra. Preferably the compositions are administered by the oral or nasal respiratory route for local or systemic effect. Compositions in preferably pharmaceutically acceptable solvents may be nebulized by use

of inert gases. Nebulized solutions may be breathed directly from the nebulizing device or the nebulizing device may be attached to a face mask, tent, or intermittent positive pressure breathing machine. Solution, suspension, or powder compositions may be administered, preferably orally or nasally, from devices which deliver the formulation in an appropriate manner.

The following formulation examples are illustrative only and are not intended to limit the scope of the invention in any way. "Active ingredient," of course, means a compound according to Formula I or a pharmaceutically acceptable salt or solvate thereof.

Formulation Example 1

Hard gelatin capsules containing the following ingredients are prepared:

| Ingredient | Quantity (mg/capsule) |
|---|---|
| Active Ingredient(s) | 30.0 |
| Starch | 305.0 |
| Magnesium stearate | 5.0 |

The above ingredients are mixed and filled into hard gelatin capsules in 340 mg quantities.

Formulation Example 2

A tablet formula is prepared using the ingredients below:

| Ingredient | Quantity (mg/tablet) |
|---|---|
| Active Ingredient(s) | 25.0 |
| Cellulose, microcrystalline | 200.0 |
| Colloidal silicon dioxide | 10.0 |
| Stearic acid | 5.0 |

The components are blended and compressed to form tablets, each weighing 240 mg.

Formulation Example 3

A dry powder inhaler formulation is prepared containing the following components:

| Ingredient | Weight % |
|---|---|
| Active Ingredient(s) | 5 |
| Lactose | 95 |

The active mixture is mixed with the lactose and the mixture is added to a dry powder inhaling appliance.

Formulation Example 4

Tablets, each containing 30 mg of active ingredient, are prepared as follows:

| Ingredient | Quantity (mg/tablet) |
|---|---|
| Active Ingredient(s) | 30.0 mg |
| Starch | 45.0 mg |
| Microcrystalline cellulose | 35.0 mg |
| Polyvinylpyrrolidone (as 10% solution in water) | 4.0 mg |
| Sodium carboxymethyl starch | 4.5 mg |
| Magnesium stearate | 0.5 mg |
| Talc | 1.0 mg |
| Total | 120 mg |

The active ingredient, starch and cellulose are passed through a No. 20 mesh U.S. sieve and mixed thoroughly. The solution of polyvinylpyrrolidone is mixed with the resultant powders, which are then passed through a 16 mesh U. S. sieve. The granules so produced are dried at 50-60°C and passed through a 16 mesh U.S. sieve. The sodium carboxymethyl starch, magnesium stearate, and talc, previously passed through a No. 30 mesh U.S. sieve, are then added to the granules which, after mixing, are compressed on a tablet machine to yield tablets each weighing 120 mg.

Formulation Example 5

Capsules, each containing 40 mg of medicament are made as follows:

| Ingredient | Quantity (mg/capsule) |
|---|---|
| Active Ingredient (s) | 40.0 mg |
| Starch | 109.0 mg |
| Magnesium stearate | 1.0 mg |
| Total | 150.0 mg |

The active ingredient, cellulose, starch, and magnesium stearate are blended, passed through a No. 20 mesh U. S. sieve, and filled into hard gelatin capsules in 150 mg quantities.

Formulation Example 6

Suppositories, each containing 25 mg of active ingredient are made as follows:

| Ingredient | Amount |
|---|---|
| Active Ingredient (s) | 25 mg |
| Saturated fatty acid glycerides to | 2,000 mg |

The active ingredient is passed through a No. 60 mesh U.S. sieve and suspended in the saturated fatty acid glycerides previously melted using the minimum heat necessary. The mixture is then poured into a suppository mold of nominal 2.0 g capacity and allowed to cool.

Formulation Example 7

Suspensions, each containing 50 mg of medicament per 5.0 ml dose are made as follows:

| Ingredient | Amount |
|---|---|
| Active Ingredient (s) | 50.0 mg |
| Xanthan gum | 4.0 mg |
| Sodium carboxymethyl cellulose (11%) | |
| Microcrystalline cellulose (89 %) | 50.0 mg |
| Sucrose | 1.75 g |
| Sodium benzoate | 10.0 mg |
| Flavor and Color | q.v. |
| Purified water to | 5.0 ml |

The medicament, sucrose and xanthan gum are blended, passed through a No. 10 mesh U.S. sieve, and then mixed with a previously made solution of the microcrystalline cellulose and sodium carboxymethyl cellulose in water. The sodium benzoate, flavor, and color are diluted with some of the water and added with stirring. Sufficient water is then added to produce the required volume.

Formulation Example 8

Capsules, each containing 15 mg of medicament, are made as follows:

| Ingredient | Quantity (mg/capsule) |
| --- | --- |
| Active Ingredient (s) | 15.0 mg |
| Starch | 407.0 mg |
| Magnesium stearate | 3.0 mg |
| Total | 425.0 mg |

The active ingredient, cellulose, starch, and magnesium stearate are blended, passed through a No. 20 mesh U. S. sieve, and filled into hard gelatin capsules in 425 mg quantities.

Formulation Example 9

An intravenous formulation may be prepared as follows:

| Ingredient | Quantity |
| --- | --- |
| Active Ingredient (s) | 250.0 mg |
| Isotonic saline | 1000 ml |

Formulation Example 10

A topical formulation may be prepared as follows:

| Ingredient | Quantity |
| --- | --- |
| Active Ingredient (s) | 1-10 g |
| Emulsifying Wax | 30 g |
| Liquid Paraffin | 20 g |
| White Soft Paraffin | to 100 g |

The white soft paraffin is heated until molten. The liquid praffin and emulsifying wax are incorporated and stirred until dissolved. The active ingredient is added and stirring is continued until dispersed. The mixture is then cooled until solid.

Formulation Example 11

Sublingual or buccal tablets, each containing 10 mg of active ingredient, may be prepared as follows:

| Ingredient | Quantity Per Tablet |
| --- | --- |
| Active Ingredient (s) | 10.0 mg |
| Glycerol | 210.5 mg |
| Water | 143.0 mg |
| Sodium Citrate | 4.5 mg |
| Polyvinyl Alcohol | 26.5 mg |
| Polyvinylpyrrolidone | 15.5 mg |
| Total | 410.0 mg |

The glycerol, water, sodium citrate, polyvinyl alcohol, and polyvinylpyrrolidone are admixed together by continuous stirring and maintaining the temperature at about 90°C. When the polymers have gone into solution, the solution is cooled to about 50-55°C and the medicament is slowly admixed. The homogenous mixture is poured into forms made of an inert material to produce a drug-containing diffusion matrix having a thickness of about 2-4 mm. This diffusion matrix is then cut to form individual tablets having the appropriate size.

Another preferred formulation employed in the methods of the present invention employs transdermal delivery devices ("patches"). Such transdermal patches may be used to provide continuous or discontinuous infusion of the compounds of the present invention in controlled amounts. The construction and use of transdermal patches for the delivery of pharmaceutical agents is well known in the art. See, e.g., U.S. Patent 5,023,252, issued June 11, 1991,

herein incorporated by reference. Such patches may be constructed for continuous, pulsatile, or on demand delivery of pharmaceutical agents.

Frequently, it will be desirable or necessary to introduce the pharmaceutical composition to the brain, either directly or indirectly. Direct techniques usually involve placement of a drug delivery catheter into the host's ventricular system to bypass the blood-brain barrier. One such implantable delivery system, used for the transport of biological factors to specific anatomical regions of the body, is described in U.S. Patent 5,011,472, issued April 30, 1991.

Indirect techniques, which are generally preferred, usually involve formulating the compositions to provide for drug latentiation by the conversion of hydrophilic drugs into lipid-soluble drugs or prodrugs. Latentiation is generally achieved through blocking of the hydroxy, carbonyl, sulfate, and primary amine groups present on the drug to render the drug more lipid soluble and amenable to transportation across the blood-brain barrier. Alternatively, the delivery of hydrophilic drugs may be enhanced by intra-arterial infusion of hypertonic solutions which can transiently open the blood-brain barrier.

## Claims

**1.** A method of reversing multidrug resistance in a multidrug resistant tumor in a mammal which comprises administering to a mammal in need thereof a multidrug resistance reversing amount of a compound of the formula

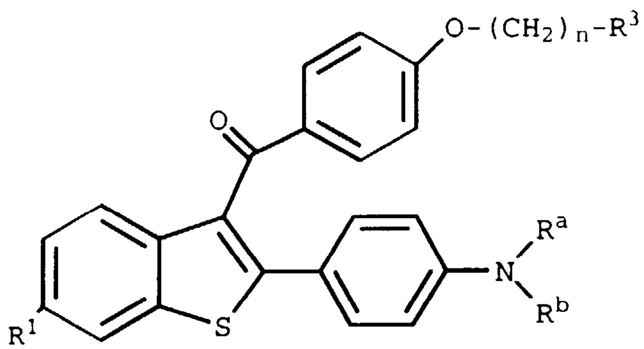

wherein:

$R^1$ is hydroxy, halo, hydrogen, $C_1$-$C_6$ alkoxy, or $C_1$-$C_6$ alkyl;

$R^a$ and $R^b$ are independently hydrogen, $C_1$-$C_6$ alkyl, -C(O)$R^c$, or -$SO_2$-$R^c$ , where $R^c$ is $C_1$-$C_6$ alkyl, halo, or trifluoromethyl;

n is 1-6;

$R^3$ is a group of the formula

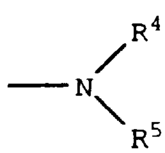

wherein $R^4$ and $R^5$ are independently $C_1$-$C_6$ alkyl or combine to form, along with the nitrogen to which they are attached, a heterocyclic ring selected from the group consisting of hexamethyleneiminyl, piperazinyl, heptamethyleneiminyl, imidazolinyl, piperidinyl, pyrrolidinyl, or morpholinyl;

or a pharmaceutically acceptable salt or solvate thereof.

**2.** A method of treating a susceptible neoplasm in a mammal which comprises administering to a mammal in need

thereof an effective amount of a compound of the formula

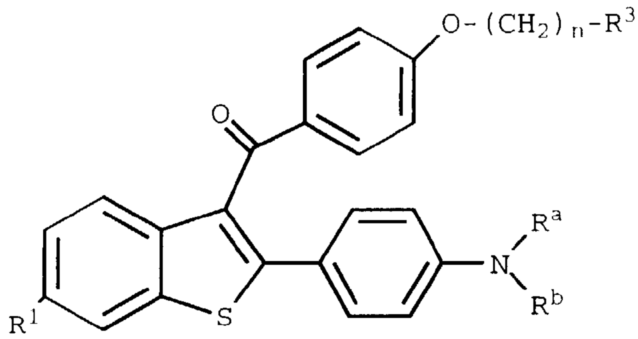

wherein:

$R^1$ is hydroxy, halo, hydrogen, $C_1$-$C_6$ alkoxy, or $C_1$-$C_6$ alkyl;

$R^a$ and $R^b$ are independently hydrogen, $C_1$-$C_6$ alkyl, -C(O)$R^c$, or -$SO_2$-$R^c$ , where $R^c$ is $C_1$-$C_6$ alkyl, halo, or trifluoromethyl;

n is 1-6;

$R^3$ is a group of the formula

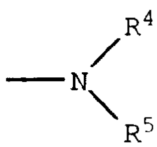

wherein $R^4$ and $R^5$ are independently $C_1$-$C_6$ alkyl or combine to form, along with the nitrogen to which they are attached, a heterocyclic ring selected from the group consisting of hexamethyleneiminyl, piperazinyl, heptamethyleneiminyl, imidazolinyl, piperidinyl, pyrrolidinyl, or morpholinyl;

or a pharmaceutically acceptable salt or solvate thereof. in combination with one or more oncolytic agents.

**3.** A compound of the formula

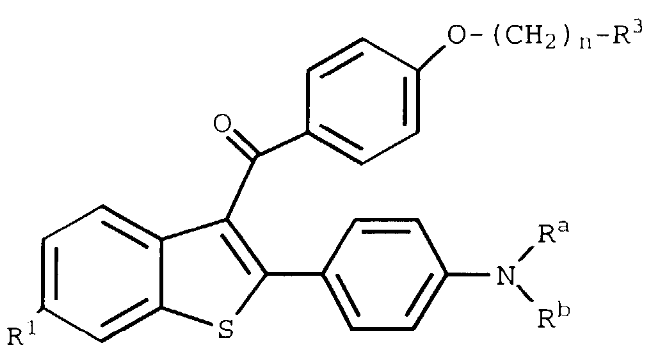

wherein:

$R^1$ is hydroxy, halo, hydrogen, $C_1$-$C_6$ alkoxy, or $C_1$-$C_6$ alkyl;

$R^a$ and $R^b$ are independently hydrogen, $C_1$-$C_6$ alkyl, -C(O)$R^c$, or -$SO_2$-$R^c$, where $R^c$ is $C_1$-$C_6$ alkyl, halo, or trifluoromethyl;

n is 1-6;

$R^3$ is a group of the formula

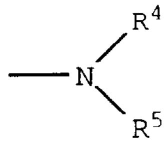

wherein $R^4$ and $R^5$ are independently $C_1$-$C_6$ alkyl or combine to form, along with the nitrogen to which they are attached, a heterocyclic ring selected from the group consisting of hexamethyleneiminyl, piperazinyl, heptamethyleneiminyl, imidazolinyl, piperidinyl, pyrrolidinyl, or morpholinyl;

or a salt or solvate thereof.

**4.** A pharmaceutical formulation comprising a compound of the formula

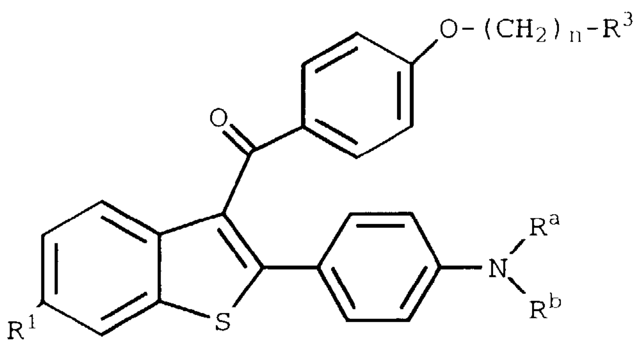

wherein:

$R^1$ is hydroxy, halo, hydrogen, $C_1$-$C_6$ alkoxy, or $C_1$-$C_6$ alkyl;

$R^a$ and $R^b$ are independently hydrogen, $C_1$-$C_6$ alkyl, -C(O)$R^c$, or -$SO_2$-$R^c$, where $R^c$ is $C_1$-$C_6$ alkyl, halo, or trifluoromethyl;

n is 1-6;

$R^3$ is a group of the formula

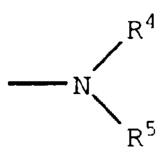

wherein $R^4$ and $R^5$ are independently $C_1$-$C_6$ alkyl or combine to form, along with the nitrogen to which they are attached, a heterocyclic ring selected from the group consisting of hexamethyleneiminyl, piperazinyl, heptamethyleneiminyl, imidazolinyl, piperidinyl, pyrrolidinyl, or morpholinyl;

or a pharmaceutically acceptable salt or solvate thereof, in combination with one or more pharmaceutically acceptable carriers, diluents, or excipients therefor.

**5.** A pharmaceutical formulation comprising:

(a) a compound of the formula

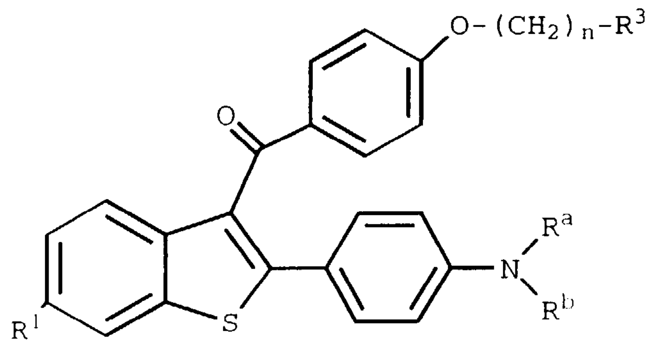

wherein:

$R^1$ is hydroxy, halo, hydrogen, $C_1$-$C_6$ alkoxy, or $C_1$-$C_6$ alkyl;

$R^a$ and $R^b$ are independently hydrogen, $C_1$-$C_6$ alkyl, -C(O)$R^c$, or -$SO_2$-$R^c$, where $R^c$ is $C_1$-$C_6$ alkyl, halo, or trifluoromethyl;

n is 1-6;

$R^3$ is a group of the formula

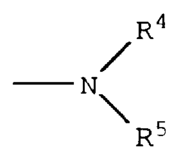

wherein $R^4$ and $R^5$ are independently $C_1$-$C_6$ alkyl or combine to form, along with the nitrogen to which they are attached, a heterocyclic ring selected from the group consisting of hexamethyleneiminyl, piperazinyl, heptamethyleneiminyl, imidazolinyl, piperidinyl, pyrrolidinyl, or morpholinyl;

or a pharmaceutically acceptable salt or solvate thereof;

(b) one or more oncolytic agents; and

(c) one or more pharmaceutically acceptable carriers, diluents, or excipients therefor.

**6.** The use of a compound of Claim 3 for the manufacture of a medicament for the treatment of multidrug resistance in a multidrug resistant tumor in a mammal.

**7.** The use of a compound of Claim 3 and one or more oncolytic agents for the manufacture of a medicament for the treatment of a susceptible neoplasm in a mammal.

**8.** A derivative of benzo[b]thiophene compound as hereinbefore described with reference to any of the examples.

European Patent Office

# EUROPEAN SEARCH REPORT

Application Number
EP 96 30 7955

## DOCUMENTS CONSIDERED TO BE RELEVANT

| Category | Citation of document with indication, where appropriate, of relevant passages | Relevant to claim | CLASSIFICATION OF THE APPLICATION (Int.Cl.6) |
|---|---|---|---|
| Y | EP 0 652 004 A (LILLY CO ELI) 10 May 1995<br>* the whole document *<br>--- | 1-8 | C07D333/56<br>A61K31/34<br>A61K31/38<br>A61K31/40 |
| Y | EP 0 617 030 A (LILLY CO ELI) 28 September 1994<br>* claims 1-7 *<br>--- | 3-5,8 | |
| P,Y | EP 0 709 090 A (LILLY CO ELI) 1 May 1996<br>* claims 1-8 *<br>--- | 1,2,5-8 | |
| D,P,A | EP 0 739 890 A (LILLY CO ELI) 30 October 1996<br>* the whole document *<br>----- | 3 | |

TECHNICAL FIELDS SEARCHED (Int.Cl.6)
C07D
A61K

The present search report has been drawn up for all claims

| Place of search | Date of completion of the search | Examiner |
|---|---|---|
| MUNICH | 14 February 1997 | Foerster, W |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)